# EUROPEAN PATENT SPECIFICATION

(11) **EP 2 706 066 B1**
(45) Date of publication and mention of the grant of the patent: **29.03.2017**
(21) Application number: 13183575.3
(22) Date of filing: 09.09.2013
(51) Int. Cl.: C25B 3/04, C07K 1/113

(54) **Electrochemical reduction of disulfide bonds in proteinaceous substances and electrochemical cell for carrying out such reduction**
Elektrochemische Reduktion von Disulfidbindungen bei proteinhaltigen Substanzen und elektrochemische Zelle zur Durchführung solch einer Reduktion
Réduction électrochimique de liaisons de disulfure dans des substances protéiques et cellule électrochimique permettant de mettre en oeuvre une telle réduction

(30) Priority: 10.09.2012 US 201213608907
(43) Date of publication of application: 12.03.2014
(73) Proprietor: Antec Leyden B.V., 2382 NV Zoeterwoude (NL)
(72) Inventor: Kraj, Agnieszka Urszula, 2341 PL Oegstgeest (NL); Brouwer, Hendrik-Jan, 2353 WL Leiderdorp (NL); Chervet, Jean-Pierre, 1017 CC Amsterdam (NL)
(74) Representative: Nederlandsch Octrooibureau

(56) References cited:
- WO-A1-2006/102722
- GB-A- 2 291 887
- ZHANG YUN ET AL: "Electrochemistry-assisted top-down characterization of disulfide-containing proteins.", ANALYTICAL CHEMISTRY 17 APR 2012, vol. 84, no. 8, 17 April 2012 (2012-04-17) , pages 3838-3842, XP002717566, ISSN: 1520-6882 & Yun Zhang: "Electrochemistry-assisted top-down characterization of disulfide-containig proteins- SUPPORTING INFORMATION-", , 26 March 2012 (2012-03-26), pages 1-5, XP055092061, Retrieved from the Internet: URL:http://pubs.acs.org/doi/suppl/10.1021/ ac300106y/suppl_file/ac300106y_si_001.pdf [retrieved on 2013-12-06]
- Jean-Pierre Chervet ET AL: "Enhanced Protein/Peptide Characterization Using Electrochemically Assisted Disulfide Bond Reduction", , 3 June 2013 (2013-06-03), pages 1-1, XP055091986, Retrieved from the Internet: URL:http://www.myantec.com/downloads/prese nted/ASMS2013/317_Chervet_Reduction_ASMS20 13.pdf [retrieved on 2013-12-06]
- Agnieszka Kraj ET AL: "Enhanced Peptide/Protein Characterization Using Electrochemically Assisted Disulfide Bond Reduction", , 20 June 2013 (2013-06-20), pages 1-1, XP055091988, Retrieved from the Internet: URL:http://www.myantec.com/downloads/prese nted/HPLC2013/221_095_01 - Electrochemically Assisted Reduction of Disulfide Bonds of Peptides and Proteins.pdf [retrieved on 2013-12-06]
- AGNIESZKA KRAJ ET AL: "A novel electrochemical method for efficient reduction of disulfide bonds in peptides and proteins prior to MS detection", ANALYTICAL AND BIOANALYTICAL CHEMISTRY, vol. 405, no. 29, 29 September 2013 (2013-09-29), pages 9311-9320, XP055091992, ISSN: 1618-2642, DOI: 10.1007/s00216-013-7374-3

## Description

### TECHNICAL FIELD OF THE INVENTION

The present invention relates to an analytical method involving the cleaving disulfide bonds in proteinaceous substances by means of electrochemical reduction, said method comprising subjecting a liquid sample containing one or more proteinaceous substances to a reducing potential in an electrochemical cell to reduce disulfide bonds in the one or more proteinaceous substances and further processing the electrochemically reduced liquid sample in an on-line fashion, e.g. by subjecting it to structure elucidation analysis.

The invention also provides a device comprising an electrochemical flow cell for carrying out the aforementioned method.

### BACKGROUND OF THE INVENTION

Disulfide bonds are important for the stabilization of the native structures of proteins. Redox-active disulfide bonds are one of the most common protein post-translational modifications (PTM) and provide reversible covalent cross-linkages in native proteins for maintaining the three-dimensional structures of proteins and their biological activities. Such a linkage plays a critical role in the activity of enzymes and is also a key structural feature of biologically active peptide hormones such as somatostatin, oxytocin, and [Arg8]-vasopressin. The presence of the disulfide linkages increases the complexity for the protein structure elucidation by, for example, mass spectrometry. The cleavage of disulfide bonds is often essential for the protein/peptide analysis as dissociation of a reduced protein/peptide ion can give rise to more structurally informative fragment ions than that of the intact counterpart.

The traditional protocol to break a disulfide bond is chemical reduction using an excess amount of reagents like dithiothreitol or tris(2-carboxyethyl)phosphine. However, this type of reduction is very laborious. Besides chemical reduction, other novel approaches include the cleavage of disulfide bonds via laser-based ionization, ultraviolet photodissociation, negative ion dissociation, electron-capture dissociation (ECD, electron-transfer dissociation (ETD),plasma-induced oxidation, reactive electrospray-assisted laser desorption/ionization (ELDI), or using new ion chemistry.

An alternative way for reducing disulfide bonds without involving chemical reductants is electrochemical reduction. It is well-known that disulfide bonds can undergo reductive cleavage on an amalgam electrode surface.

Zhang et al. (Online Mass Spectrometric Analysis of Proteins/Peptides Following Electrolytic Cleavage of Disulfide Bonds, J. Proteome Res. 2011, 10, 1293-1304) report the results of a study in which structural analysis of biologically active peptides and proteins containing disulfide bonds using
electrochemistry (EC) online was combined with desorption electrospray ionization mass spectrometry (DESI-MS), and in which the sample undergoes electrolytic disulfide cleavage in an electrochemical flow cell followed by MS detection. Using this EC/DESI-MS method, the disulfide-containing peptides
could be quickly identified from enzymatic digestion mixtures, simply based on the abrupt decrease in their relative ion abundances after electrolysis. The authors employed a home-built apparatus for coupling a thin-layer electrochemical flow cell with a mass spectrometer by liquid sample DESI. The thin-layer electrochemical flow cell consisted of a working electrode embedded in PEEK and separated from a stainless steel auxiliary electrode by two Teflon gaskets and a Ag/AgCl (3 M NaCl) reference electrode contacting the sample solution through a small hole in the auxiliary electrode. The working electrode used was a dual amalgam electrode (3 mm diameter) or an amalgam electrode (6mm diameter) for reduction.

Zhang et al. (Electrochemistry-Assisted Top-Down Characterization of Disulfide-Containing Proteins, Anal. Chem. 2012, 84, 3838-3842) describe electrochemistry-assisted top-down characterization of disulfide-containing proteins by integrating electrochemistry (EC) online with a top-down MS approach. In this approach, proteins undergo electrolytic reduction in an electrochemical cell to break disulfide bonds and then undergo online ionization into gaseous ions for analysis by electron-capture dissociation (ECD) and collision-induced dissociation (CID). The authors conclude that electrochemical reduction of proteins allows one to remove disulfide bond constraints and also leads to increased charge numbers of the resulting protein ions. As a result, sequence coverage was significantly enhanced, as exemplified by β-lactoglobulin A (24 vs 75 backbone cleavages before and after electrochemical reduction, respectively) and lysozyme (5 vs 66 backbone cleavages before and after electrochemical reduction, respectively).

Lu et al. (Investigation of some biologically relevant redox reactions using electrochemical mass spectrometry interfaced by desorption electrospray ionization, .Analytical Bioanalytical Chemistry, 2012, 403, 345-355) have employed an EC/DESI-MS method to investigate some aqueous phase redox reactions, including the reduction of peptide disulfide bonds. It was found that knotted/enclosed disulfide bonds in the peptides apamin and endothelin could be ectrochemically cleaved. A thin-layer electrochemical flow cell equipped with a boron-doped diamond was employed for the peptide reduction experiments.

WO 2006/102722 A1 describes a process for preparing a compound comprising an alpha,gamma amide linkage between a cysteine moiety and a glutamic acid moiety, comprising providing a cysteine derivative, a gamma-glutamyl donor and an enzyme capable of transferring the gamma-glutamyl group to said cysteine derivative in a reaction environment promoting transfer of the gamma- glutamyl group to said cysteine derivative. Example 12 describes electrochemical reduction of gamma-Glutamylcysteine-mercaptoethanol (GGC-Merc) disulphide. Batch electrolyses were performed with a lead cathode and a catholyte (200 mL) containing up to 0.2 M GGC-Merc disulphide. The anode was platinised-titanium and the anolyte (200 mL) was 2 M sulphuric acid.

GB 2,291,887 A relates to a method of the electrolytic reduction of a disulfide compound. More particularly, this patent application describes the use of a titanium-containing cathode in the production of cystein by electrolytic reduction of cystin.

### SUMMARY OF THE INVENTION

The inventors have developed a substantially improved method for cleaving disulfide bonds in proteinaceous substances by means of electrochemical reduction. More particularly, the inventors have found that fast, stable and substantially complete reduction of all disulfide bonds in proteinaceous substances can be achieved by employing a working electrode that contains titanium.

Thus, the present invention concerns an analytical method involving the cleaving disulfide bonds in proteinaceous substances by means of electrochemical reduction, said method comprising:
- providing a liquid sample containing one or more proteinaceous substances that comprises at least one disulfide bond;
- providing an electrochemical cell comprising a working electrode and an auxiliary electrode;
- introducing the liquid sample into the electrochemical cell;
- subjecting the liquid sample in the electrochemical cell to a reducing potential to reduce disulfide bonds in the one or more proteinaceous substances, thereby producing an electrochemically reduced liquid sample containing proteinaceous substances with a cleaved disulfide bond; and
- detection analysis or structure elucidation analysis of the electrochemically reduced liquid sample in an on-line fashion;
wherein the electrochemical cell comprises a working electrode that contains titanium

The invention further relates to a device for processing and analyzing a sample fluid, the device comprising an electrochemical flow cell comprising:
- a body having a flow path, the flow path having an inlet and an outlet;
- a working electrode in fluid communication with the flow path; and
- an auxiliary electrode;
wherein the working electrode comprises a component or layer that contains at least 20 wt.% titanium in the form of elemental titanium, titanium-containing substances and/or titanium-containing alloys; and wherein the outlet of the cell is connected to an electrochemical detection (ECD) device, a NMR spectrometer or a mass spectrometer (MS).

An electrochemical cell comprising a working electrode that contain titanium is described by Wu et al. (Fabrication of electrolytic cell for online post-column electrochemical derivatization in ion chromatography, Analytica Chimica Acta 735 (2012) 62- 68). The home-made electrochemical cell described by Wu et al. consisted of two quadrate PTFE blocks placed on top of the other in a symmetrical structure separated by two pieces of cation-exchange membranes. A Ru/Ti electrode (49.6 mm × 10 m × 0.5 mm, provided by Qingdao Shenghan Chromatography Tech Equipment Co., LTD., Qingdao, China) was embedded into the chamber in each block. Each electrode had two holes (0.8 mm in diameter and about 3 mm away from each end of the electrode). The holes were drilled as the inlet and outlet for the solution, respectively. Cation exchange resins were filled in the space between each electrode and CEM to serve as ion conductors. The solution flowed through the gaps among the resins.

As demonstrated by the inventors, the present invention enables on-line disulfide bond reduction and can be used for the determination of disulfide bond arrangements in top down proteomics strategies (using an EC/DESI-MS configuration).

The rapid reduction of the disulfide bonds, allows for on-line coupling with HPLC in either pre- or post-column mode.

### DETAILED DESCRIPTION OF THE INVENTION

Accordingly, a first aspect of the inventions relates to an analytical method involving the cleaving disulfide bonds in proteinaceous substances by means of electrochemical reduction, said method comprising:
- providing a liquid sample containing one or more proteinaceous substances that comprises at least one disulfide bond;
- providing an electrochemical cell comprising a working electrode and an auxiliary electrode;
- introducing the liquid sample into the electrochemical cell;
- subjecting the liquid sample in the electrochemical cell to a reducing potential to reduce disulfide bonds in the one or more proteinaceous substances, thereby producing an electrochemically reduced liquid sample containing proteinaceous substances with a cleaved disulfide bond; and
- detection analysis or structure elucidation analysis of the electrochemically reduced liquid sample in an on-line fashion;
wherein the electrochemical cell comprises a working electrode that contains titanium

The term "proteinaceous substance" as used herein refers to a substance comprising a linear polymer chain of at least 3 amino acids bonded together by peptide bonds

The term "proteinaceous substance comprising at least one disulfide bond" refers to a proteinaceous substance comprising a covalent bond derived by the coupling of two thiol groups. This linkage is also called an SS-bond or disulfide bridge. Disulfide bonds are usually formed from the oxidation of thiol groups. The transformation is depicted as follows: 2 RSH → RS-SR + 2 H+ + 2 e-. Alternatively, disulfide bonds in proteins can be formed by thiol-disulfide exchange: RS-SR + R'SH R'S-SR + RSH.

The disulfide bonds in the proteinaceous substances can occur as inter-chain bonds as well as intra-chain bonds. Somatostatin is an example of a proteinacous substance that contains one intrachain disulfide bond that maintains the cyclic structure. Insulin is an example of a proteinaceous substance that contains wo interchain disulfide bonds and one intrachain disulfide bond.

The term "proteinaceous substance with a cleaved disulfide bond" refers to a proteinaceous substance comprising one or more pairs of thiol groups that formed a disulfide bond prior to the subjecting of the liquid sample to the reducing potential.

The term "reduction" as used herein refers to the gain of electrons / hydrogen or loss of oxygen / decrease in oxidation state by a molecule, atom or ion.

The term "oxidation" as used herein refers to the loss of electrons / hydrogen or the gain of oxygen / increase in oxidation state by a molecule, atom or ion.

The terminology "electrochemical reduction" as used herein refers to a reduction reaction that occurs in, or is initiated in a solution at the interface of an electron conductor (an electrode) and an ionic conductor (the electrolyte), and which is driven by an external applied voltage.

The terminology "electrochemical oxidation" as used herein refers to an oxidation reaction that occurs in, or is initiated in a solution at the interface of an electron conductor (an electrode) and an ionic conductor (the electrolyte), and which is driven by an external applied voltage.
Whenever reference is made herein to an electrical potential, unless indicated otherwise, said potential is defined relative to a Pd/H₂ reference electrode.

The term "electrochemical flow cell" as used herein refers to a cell in which a voltage can be applied to a fluid passing through said cell to oxidize or reduce electrochemically active substances that are contained in the fluid.

The proteinaceous substances whose disulfide bonds are cleaved in the present method are typically selected from the group consisting of proteins, peptides, glycoproteins, phosphoproteins, ubiquitinated proteins and combinations thereof. Preferably, the proteinaceous substances contain at least 6 amino acid residues.

The liquid sample typically contains 5 ng/ml to 5 mg/ml, more preferably 50 ng/ml to 500µg/ml of the one or more proteinaceous substances.

The liquid samples employed in the present method advantageously contain a solvent, preferably a solvent selected from water, organic solvents and combinations thereof. Preferably, solvent is contained in these samples in a sufficient amount to render these samples liquid and should contain a sufficient amount of electrolyte. Typically, the samples contain at least 95 wt.% of solvent, most preferably at least 98 wt.% of solvent.

In the present method, the reducing potential applied typically is in the range of -0.1 V to -10 V, more preferably in the range of -0.5 to -6 V and most preferably in the range of -1 V to -3 V.

The working electrode employed in the present method preferably contains at least 20 wt.%, more preferably at least 40 wt.% and most preferably at least 50 wt.% titanium in the form of elemental titanium, titanium-containing substances and/or titanium-containing alloys.

In accordance with a preferred embodiment of the present invention the electrochemical cell is an electrochemical flow cell. The use of an electrochemical flow cell offers the advantage that very small sample volumes can be handled and that 'carry-over' effects can be minimized very effectively.

Particularly good results can be obtained with the present method using a sample flow rate in the electrochemical cell that lies within the range of 10 nl/min to 3.0 ml/min. More preferably, said flow rate lies within the range of 30 nl/min to 1.0 ml/min, more preferably 0.1-100 µl/min and most preferably of 1-50 µl/min.

The residence time of the liquid sample in the electrochemical cell preferably is in the range of 1 second to 5 minutes, more preferably in the range of 2 to 60 seconds, most preferably in the range of 8 to 20 seconds.

In the present method the reducing potential is maintained during a period of at least 0.02 seconds, more preferably during a period of at least 0.1 seconds and most preferably during a period of at least 0.3 seconds.

According to a particularly preferred embodiment the reducing potential is applied during discrete time intervals that alternate with time intervals during which an oxidizing potential is applied. By alternating pulses of reducing voltage with pulses of oxidizing voltage very efficient cleavage of the disulfide bonds in the proteinaceous substances can be achieved. Although the inventors do not wish to be bound by theory it is believed that a stable layer of metal dioxide is generated by the anodic pulse on the pure metal working electrode surface and that this layer of metal dioxide contributes to the efficient reduction of disulfide bonds. Furthermore, by applying pulses of reductive and oxidative potentials may achieve continuous renewal of the electrode surface and thereby prevent adsorption of proteins/peptides.

The oxidizing potential that is applied during the intervals between successive reducing pulses advantageously is in the range of 0.05 V to 10 V, more preferably in the range of 0.3 V to 6 V and most preferably in the range of 0.5 V to 1.0 V.

The discrete time intervals during which the reducing potential is applied typically have a duration of 0.1 to 300 seconds, more preferably of 0.3 to 60 seconds.

It is preferred that the time period between the discrete time intervals during which the reducing potential is applied is shorter than the residence time of the liquid sample in the electrochemical cell. Typically, the time period between the discrete time intervals during which the reducing potential is applied is in the range of 0.1-300 seconds, more preferably in the range of 0.2-20 seconds and most preferably in the range of 0.3-2.0 seconds.

The duration of the interval during which an oxidizing potential is applied typically lies in the range of 0.1 to 300 seconds, more preferably of 0.2 to 20 seconds and most preferably of 0.3 to 2.0 seconds.

Analytical techniques that may suitably be used to detect proteinaceous substances with a cleaved disulfide bond include electrochemical detection (ECD) devices, e.g. using an Au electrode.

Analytical techniques that can suitably be used for structure elucidation included mass spectrometry and NMR.

The present method enables substantially complete reduction of the redox-active disulfide bonds in the proteinaceous substances. Typically, at least 90 % of the disulfide bonds in the one or more proteinaceous substances are reduced in the electrochemical cell.

Another aspect of the invention relates to a device for processing and analyzing a sample fluid, the device comprising an electrochemical flow cell comprising:
- a body having a flow path, the flow path having an inlet and an outlet;
- a working electrode in fluid communication with the flow path; and
- an auxiliary electrode;
wherein the working electrode comprises a component or layer that contains at least 20 wt.% titanium in the form of elemental titanium, titanium-containing substances and/or titanium-containing alloys; and wherein the outlet of the cell is connected to an electrochemical detection (ECD) device, a NMR spectrometer or a mass spectrometer (MS).

The term "electrochemical flow cell" encompasses, for instance, electrochemical reactor cells and electrochemical conversion cells. Examples of electrochemical flow cells (or flow-through cells) that may be employed in accordance with the present invention are coulometric as well as amperometric cells, such as, for instance, thin-layer, wall-jet and porous electrode designs.

Typically, the electrochemical cell employed in accordance with the present invention has a working volume of 1.0 nl to 100 ml. More preferably, the electrochemical cell has a working volume of 5.0 nl to 100 µl, even more preferably of 10 nl to 30 µl, and most preferably of 30 nl to 15 µl.

The electrochemical cell comprises at least a working electrode and an auxiliary electrode. Preferably, the electrochemical cell further comprises a reference electrode. Although electrochemical reduction of samples can be achieved using a two-electrode system that comprises a working electrode and an auxiliary electrode, the use of a three-electrode system that additionally contains a reference electrode, offers the advantage that changes in working potential due to polarization effects can be avoided.

The working electrode that is employed in accordance with the present invention preferably comprises a component or layer (e.g. a monolayer) that contains at least 20 wt.%, more preferably at least 40 wt.% and most preferably at least 50 wt.% titanium in the form of elemental titanium, titanium-containing substances and/or titanium-containing alloys.

The working electrode of the present invention may be made solely from elemental titanium. Alternatively, the working electrode may contain a substrate element that is made from elemental titanium and that is coated with noble metal (e.g. platinum, iridium, ruthenium), with tantalum or with combinations of two or more of these metals. Alternatively, the titanium element may be coated with one or more oxides of the aforementioned metals. Furthermore, the titanium element may be coated with one or more titanium substances, notably with one or more titanium oxides.

The titanium element may also be doped with one or more of the aforementioned metals, e.g. platinum.

Examples of titanium-containing substances that may be employed include titanium oxides. Typically, the titanium-containing substances contain at least 20 wt.%, more preferably at least 40 wt.% titanium, said weight percentage being calculated on the molecular weight of the substance.

Examples of titanium-containing alloys include Ti-Pd, Ti-Ru, Ti-Al. These alloys preferably contain at least 50 wt.% titanium, more preferably at least 90 wt.% titanium.

The titanium containing component that is comprised in the working electrode typically has a weight of at least 1 mg, more preferably of at least 10 mg, even more preferably of at least 50 mg and most preferably of at least 100 mg. The weight of the titanium containing component typically does not exceed 100 g. Even more preferably, said weight does not exceed 10 g.

The electrochemical cell of the present invention preferably is a thin layer electrochemical flow cell with a working volume of 0.1 µl to 1 ml, preferably of 0.5-100 µl and a distance between the working electrode and the auxiliary electrode of 10-150 µm, more preferably of 30-120 µm and most preferably of 50-100 µm.

The auxiliary electrode that is employed in the electrochemical cell advantageously comprises an electrode material selected from titanium, graphite, platinum and combinations thereof.

As explained herein before, the reduction of the disulfide bonds in the proteinaceous substances is advantageously achieved by applying an alternating reducing and oxidizing voltage. Accordingly, it is preferred that the electrochemical cell comprises a programmable controlling unit that can be programmed to vary the applied potential according to a predetermined pattern.

The controlling unit may be arranged as a CPU, a personal computer, a server, a laptop, etc. The controlling unit comprises a processor unit performing arithmetical operations. The processor unit is connected to a memory unit that can store instructions and data, such as a hard disk, a Read Only Memory (ROM), Electrically Erasable Programmable Read Only Memory (EEPROM), a Random Access Memory (RAM), a CD, a DVD, a USB-stick or combinations thereof. The controlling device may further comprise user interface devices such as a keyboard, a mouse, a display a printer.

According to an embodiment, the controlling device is arranged to perform any one of the methods provided by the present invention. In order to do so, the memory unit may comprise instructions that are readable and executable by the processing unit. The processing unit is arranged to communicate via the input-output device to send instructions to the electrochemical flow cell.

The sample fluid may suitably be introduced into the electrochemical flow cell with the help of a syringe pump or an injection valve. The sample fluid may also be taken directly from a high pressure liquid chromatograph (HPLC). Accordingly, in a preferred embodiment of the electrochemical flow cell the inlet of the cell is connected to a HPLC, a syringe pump or an injection valve.

The device of the present invention offers the important advantage that cleavage of disulfide bonds in proteinaceous substances is achieved in such a fast and reproducible way that the cell can suitably be used as an in-line sample preparation device. The outlet of the electrochemical flow cell is connected to an analytical device that is capable of detecting proteinaceous substances with a cleaved disulfide bond or of elucidating the structure of said substances, i.e. the outlet of the cell is connected to an electrochemical detection (ECD) device, a NMR spectrometer or a mass spectrometer (MS). Even more preferably, the outlet of the cell is connected to an ECD device (especially an ECD device capable of detecting free thiol groups) or a mass spectrometer. Most preferably, the outlet of the cell is connected to a mass spectrometer.

The mass spectrometer may suitably comprises an ionization interface selected from Electrospray Ionization (ESI) and Matrix Assisted Laser Desorption Ionization (MALDI).

Most preferably, the ionization interface employed is ESI. The present apparatus offer the advantage that it can employ all types of ESI (ion spray, electrospray, nano electrospray, DESI etc.) without any modifications/adaptations.

The outlet of the electrochemical flow cell may also suitably connected to a chemical separation device. Examples of chemical separation devices that may be employed include a liquid chromatograph (including HPLC) and an electrophoresis device. Preferably, the chemical separation device is a liquid chromatograph, most preferably an HPLC (including UPLC).

Unlike the electrochemical flow cell described in the earlier mentioned reference Wu et al. (2012), the cell disclosed herein preferably is not filled with cation exchange resin. Even more preferably, the electrochemical flow cell does not contain cation exchange resin.

The invention is further illustrated by means of the following non-limiting examples.

### EXAMPLES

### Example 1

To demonstrate the performance of the titanium working electrode in the reduction of disulfide bonds in peptides and proteins prior to MS detection insulin (Bovine pancreas) was used as a model compound and the result was compared with a previous approach using conductive diamond working electrode (Y. Sun, P.C. Andrews and D.L. Smith, Journal of Protein Chemistry, 1990, 9, 151-157).

Insulin consists of 51 amino acids forming two chains, A and B, and contains 3 disulfide bonds. Two interchain disulfide bonds connect chain A and B and one intrachain disulfide bond is located on chain

### Reagents

Insulin from bovine pancreas and formic acid (99%) were obtained from Sigma Aldrich (The Netherlands).

Acetonitrile (99.9 %) was obtained from Acros organics (Belgium).

All these reagents were used as received without further purification.

Deionized water (18.2 MΩ.cm) used for all experiments was obtained from a Barnstead Easypure II system (ThermoFischer Scientific, USA).

### Methods

All experiments were performed on ROXY EC system (Antec, The Netherlands) consisting of a Potentiostat, equipped with a preparative electrochemical cell (µ - PrepCell) and an infusion pump. A boron dopped diamond (Magic Diamond, Antec, The Netherlands) or a titanium working electrode were used for reduction of disulfide bonds.

Typically, 1 - 3 µM solutions of the target compound (peptide, protein, etc.) in 1% formic acid /acetonitrile (90/10, v/v) were pumped through the electrochemical (EC) cell at a flow rate of 50 µL/min using the infusion pump. The cell was operating in pulse mode. Square wave pulses were as follows: E1=-3V(t1=1990ms) and E2=+1V (t2=1010ms), E3 and t3 were set to 0.

The inlet block of the cell was employed as counter electrode (made of titanium) and a Pd/H2 electrode (Antec). was used as reference electrode (REF). The working electrode and the auxiliary electrode inlet block were separated by a 150 µm spacer giving a cell volume of approx. 12 µL.
The ROXY EC system was controlled by Dialogue software (Antec, The Netherlands).

In EC/MS experiments a grounding union was used between the electrochemical cell and the ESI source of the LTQ - FT mass spectrometer (ThermoFisher Scientific, USA). Spectra were collected in positive mode. The mass spectrometer was calibrated following a standard optimization procedure.
The ion source parameters varied with different analytes: the spray voltage ranged between 3.2 and 3.6 kV; and the tube lens varied between 90 and 200 V. The sheath was between 15-20 units and aux gas was set between 0 and 5 units. The transfer capillary temperature was set to 275 °C. Mass spectra were recorded in full scan from 100-2000 m/z, first with the linear trap, followed by Fourier transform ion cyclotron resonance (FTICR) measurement. The data were recorded with Xcalibur software (version 2.0.7).

### Results

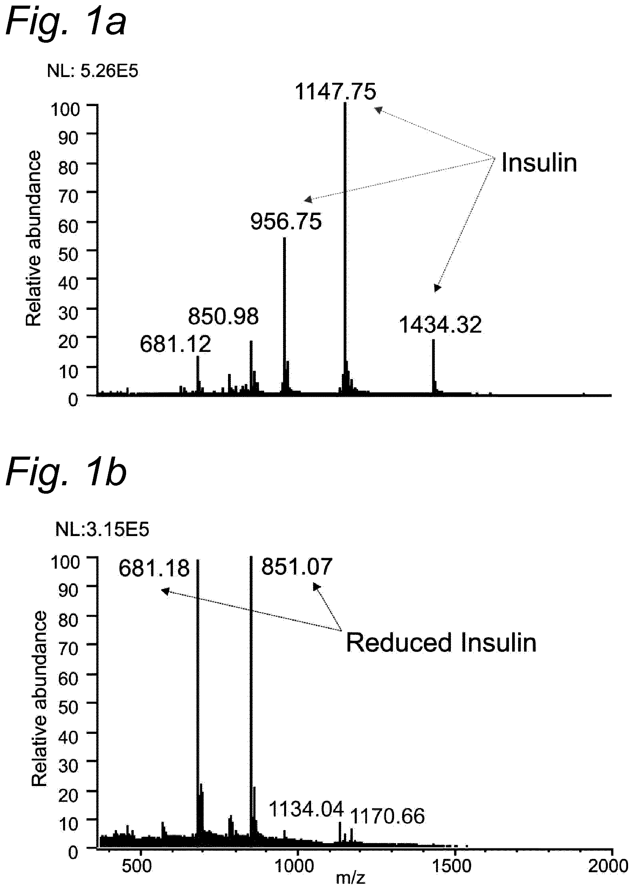

Complete reduction of insulin was observed by using the new electrode (Fig. 1) and the square wave pulses.

### Example 2

Example 1 was repeated, except that this time somatostatin was used as the test substance. Somatostatin 14 was purchased from Bachem (Switzerland).

Somatostatin, a 14 amino acids peptide is a release-inhibiting factor which has one intrachain disulfide bond that maintains the cyclic structure. Insulin is a hormone produced by the pancreas.

Table 1 summarizes the theoretical m/z values for somatostatin and its reduced forms.

**Table 1**

| | Somatostatin | Somatostatin reduced |
|---|---|---|
| [M⁺3H]3⁺ | 546.5795 | 547.2514 |
| [M⁺2H]2⁺ | 819.3656 | 820.3734 |

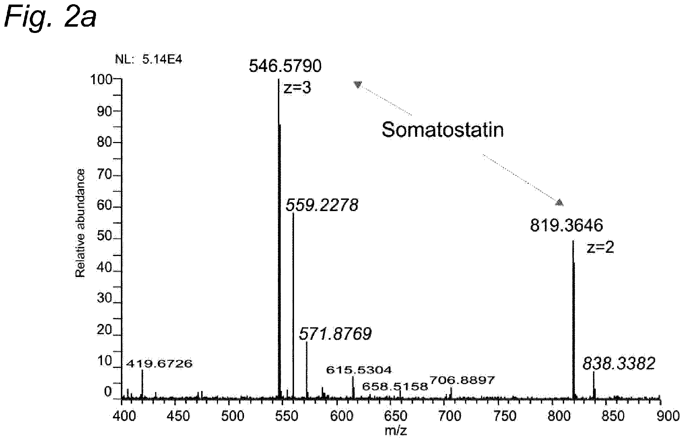

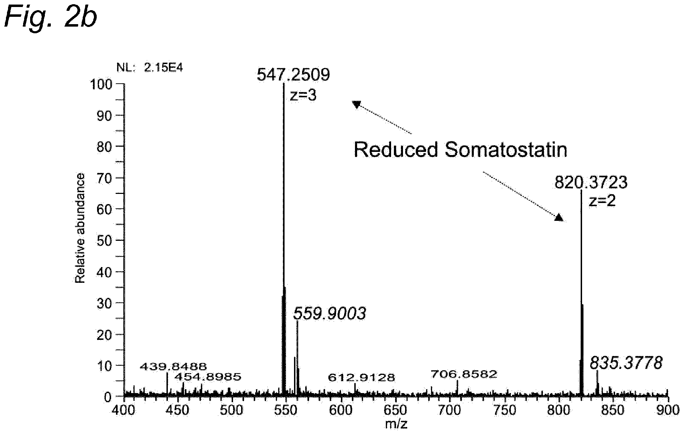

Somatostatin was completely reduced and, furthermore, a decrease in the potassium adducts was noticed (Fig. 2), a positive side effect of the electrochemical process.

### Example 3

α-lactalbumin was used as a test compound using the set-up described in Example 1 to cover a higher molecular weight protein. The α-lactalbumin from bovine milk was obtained from Sigma Aldrich (The Netherlands)

α-Lactalbumin contains 123 amino acids (counting the lactose synthase subunit). The globular structure of α-Lactalbumin is stabilized by four disulfide bonds: Cys25-Cys139, Cys47-Cys130, Cys80-Cys96, and Cys92-Cys110.

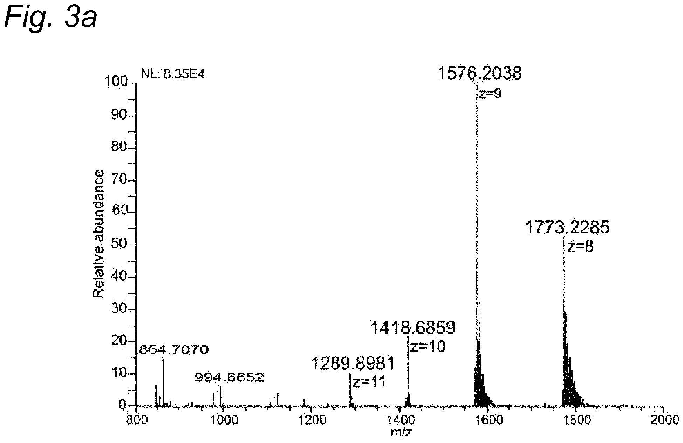

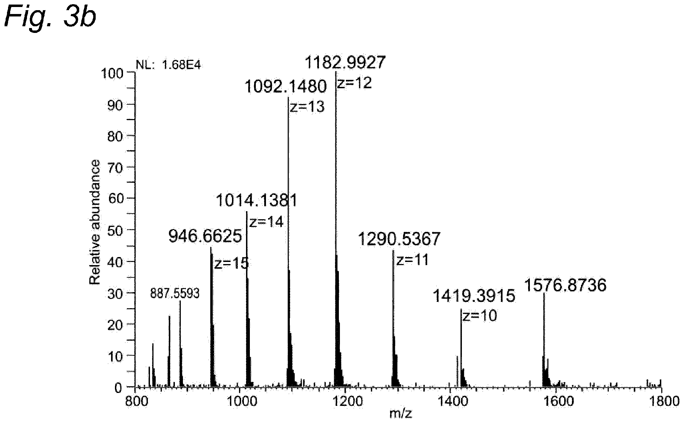

As a result of the reduction of larger proteins such as α-lactalbumin, a shift in charge state distribution was observed. This effect indicates the conformational change of the protein due to the reduction of disulfide bonds and can result in accumulation of larger number of charges on the surface of the protein (Fig. 3).

## Claims

1. A analytical method involving the cleaving disulfide bonds in proteinaceous substances by means of electrochemical reduction, said method comprising:
• providing a liquid sample containing one or more proteinaceous substances that comprises at least one disulfide bond;
• providing an electrochemical cell comprising a working electrode and an auxiliary electrode;
• introducing the liquid sample into the electrochemical cell;
• subjecting the liquid sample in the electrochemical cell to a reducing potential to reduce disulfide bonds in the one or more proteinaceous substances, thereby producing an electrochemically reduced liquid sample containing proteinaceous substances with a cleaved disulfide bond; and
• detection analysis or structure elucidation analysis of the electrochemically reduced liquid sample in an on-line fashion;
wherein the electrochemical cell comprises a working electrode that contains titanium.

2. Method according to claim 1, wherein the reducing potential applied is in the range of -0.1 V to -10 V, preferably in the range of -1 V to -3 V.

3. Method according to claim 1 or 2, wherein the electrochemical cell is an electrochemical flow cell.

4. Method according to any one of the preceding claims, wherein the reducing potential is applied during discrete time intervals that alternate with time intervals during which an oxidizing potential is applied.

5. Method according to claim 4, wherein the oxidizing potential applied is in the range of 0.05 V to 10 V, preferably in the range of 0.5 V to 1.0 V.

6. Method according to claim 4 or 5, wherein the discrete time intervals during which the reducing potential is applied have a duration of 0.1 to 300 seconds.

7. Method according to any one of the preceding claims, wherein at least 5% of the disulfide bonds in the one or more proteinaceous substances are reduced in the electrochemical cell.

8. Method according to any one of the preceding claims, wherein the working electrode comprises a component or layer that contains at least 20 wt.% titanium in the form of elemental titanium, titanium-containing substances and/or titanium-containing alloys.

9. A device for processing and analyzing a sample fluid, the device comprising an electrochemical flow cell comprising:
• a body having a flow path, the flow path having an inlet and an outlet;
• a working electrode in fluid communication with the flow path; and
• an auxiliary electrode;
wherein the working electrode comprises a component or layer that contains at least 20 wt.% titanium in the form of elemental titanium, titanium-containing substances and/or titanium-containing alloys; and
wherein the outlet of the cell is connected to an electrochemical detection (ECD) device, a NMR spectrometer or a mass spectrometer (MS).

10. Device according to claim 9, wherein the electrochemical cell is a thin layer electrochemical flow cell with a working volume of 0.1 µl to 1 ml and a distance between the working electrode and the auxiliary electrode of 10-150 µm.

11. Device according to claim 9 or 10, wherein the auxiliary electrode comprises an electrode material selected from titanium, graphite, platinum and combinations thereof.

12. Device according to any one of claims 9-11, wherein the working electrode comprises a titanium element.

## Patentansprüche

1. Analytisches Verfahren zur Spaltung von Disulfidbindungen in protein-artigen Substanzen mittels elektrochemischer Reduktion, wobei das Verfahren Folgendes aufweist:
• Bereitstellen einer flüssigen Probe, welche ein oder mehrere proteinartige Substanzen aufweist, die mindestens eine Disulfidbindung aufweist;
• Bereitstellen einer elektrochemischen Zelle, die eine Arbeitselektrode und eine Hilfselektrode aufweist;
• Einführen der flüssigen Probe in die elektrochemische Zelle;
• Aussetzen der flüssigen Probe in der elektrochemischen Zelle einem Reduktionspotential, um die Disulfidbindungen in der einen oder den mehreren proteinartigen Substanzen zu reduzieren, wodurch eine elektrochemisch reduzierte flüssige Probe erzeugt wird, welche proteinartige Substanzen aufweist mit einer gespaltenen Disulfidbindung; und
• Detektionsanalyse oder Strukturaufklärungsanalyse der elektrochemisch reduzierten Flüssigkeitsprobe in mitlaufender Weise;
wobei die elektrochemische Zelle eine Arbeitselektrode aufweist, welche Titan enthält.

2. Verfahren nach Anspruch 1, wobei das angelegte Reduktionspotential im Bereich von -0,1 V bis -10 V, vorzugsweise im Bereich von -1 V bis -3 V liegt.

3. Verfahren nach Anspruch 1 oder 2, wobei die elektrochemische Zelle eine elektrochemische Durchflusszelle ist.

4. Verfahren nach einem der vorhergehenden Ansprüche, wobei das Reduktionspotential während diskreter Zeitintervalle angelegt wird, die mit Zeitintervallen abwechseln, während denen ein Oxidationspotential angelegt wird.

5. Verfahren nach Anspruch 4, wobei das angelegte Oxidationspotential im Bereich von 0,05 V bis 10 V, vorzugsweise im Bereich von 0,5 V bis 1,0 V liegt.

6. Verfahren nach Anspruch 4 oder 5, wobei die diskreten Zeitintervalle, in denen das Reduktionspotential angelegt wird, eine Dauer von 0,1 bis 300 Sekunden haben.

7. Verfahren nach einem der vorhergehenden Ansprüche, wobei mindestens 5% der Disulfidbindungen in der einen oder den mehreren proteinartigen Substanzen in der elektrochemischen Zelle reduziert werden.

8. Verfahren nach einem der vorhergehenden Ansprüche, wobei die Arbeitselektrode eine Komponente oder Schicht aufweist, die mindestens 20 Gew.-% Titan in Form von elementarem Titan, Titan-enthaltende Substanzen und/oder titanhaltigen Legierungen enthält.

9. Vorrichtung zum Verarbeiten und Analysieren einer Probenflüssigkeit, wobei die Vorrichtung eine elektrochemische Durchflusszelle aufweist, die Folgendes aufweist:
• einen Körper mit einem Strömungspfad, wobei der Strömungspfad einen Einlass und einen Auslass besitzt;
• eine Arbeitselektrode in Fluidverbindung mit dem Strömungspfad; und
• eine Hilfselektrode;
wobei die Arbeitselektrode eine Komponente oder Schicht umfasst, die mindestens 20 Gew.-% Titan in Form von elementarem Titan, Titan-enthaltenden Substanzen und/oder titanhaltigen Legierungen enthält; und wobei der Ausgang der Zelle zu einer elektrochemischen Detektions-(ECD)-Vorrichtung, einem Kernspinresonanz- bzw. NMR-Spektrometer oder einem Massenspektrometer (MS) verbunden ist.

10. Vorrichtung nach Anspruch 9, wobei die elektrochemische Zelle eine elektrochemische Dünnschicht-Durchflusszelle mit einem Arbeitsvolumen von 0,1 µl bis 1 ml und einem Abstand zwischen der Arbeitselektrode und der Hilfselektrode von 10-150 µm ist.

11. Vorrichtung nach Anspruch 9 oder 10, wobei die Hilfselektrode ein Elektrodenmaterial aufweist, welches aus Titan, Graphit, Platin und Kombinationen davon ausgewählt ist.

12. Vorrichtung nach einem der Ansprüche 9-11, wobei die Arbeitselektrode ein Titanelement aufweist.

## Revendications

1. Méthode analytique impliquant le clivage de liaisons disulfure dans des substances protéiques par le biais d'une réduction électrochimique, ladite méthode comprenant :
• la fourniture d'un échantillon de liquide contenant une ou plusieurs substances protéiques qui comprend au moins une liaison disulfure ;
• la fourniture d'une cellule électrochimique comprenant une électrode de travail et une électrode auxiliaire ;
• l'introduction de l'échantillon de liquide dans la cellule électrochimique ;
• la soumission de l'échantillon de liquide dans la cellule électrochimique à un potentiel de réduction pour réduire les liaisons disulfure dans la ou les substances protéiques, produisant ainsi un échantillon de liquide électrochimiquement réduit contenant les substances protéiques avec une liaison disulfure clivée ; et
• l'analyse de détection ou l'analyse d'élucidation de la structure de l'échantillon de liquide électrochimiquement réduit de façon en ligne ;
dans laquelle la cellule électrochimique comprend une électrode de travail qui contient du titane.

2. Méthode selon la revendication 1, dans laquelle le potentiel de réduction appliqué est compris dans la plage allant de -0,1 V à -10 V, de préférence dans la plage allant de -1 V à -3 V.

3. Méthode selon la revendication 1 ou 2, dans laquelle la cellule électrochimique est une cellule d'écoulement électrochimique.

4. Méthode selon l'une quelconque des revendications précédentes, dans laquelle le potentiel de réduction est appliqué pendant des intervalles de temps discrets en alternance avec des intervalles de temps pendant lesquels un potentiel d'oxydation est appliqué.

5. Méthode selon la revendication 4, dans laquelle le potentiel d'oxydation appliqué est compris dans la plage allant de 0,05 V à 10 V, de préférence dans la plage allant de 0,5 V à 1,0 V.

6. Méthode selon la revendication 4 ou 5, dans laquelle les intervalles de temps discrets pendant lesquels le potentiel de réduction est appliqué ont une durée de 0,1 à 300 secondes.

7. Méthode selon l'une quelconque des revendications précédentes, dans laquelle au moins 5 % des liaisons disulfure dans la ou les substances protéiques sont réduites dans la cellule électrochimique.

8. Méthode selon l'une quelconque des revendications précédentes, dans laquelle l'électrode de travail comprend un composant ou une couche qui contient au moins 20 % en poids de titane sous la forme de l'élément titane, de substances contenant du titane et/ou d'alliages contenant du titane.

9. Dispositif pour le traitement et l'analyse d'un échantillon liquide, le dispositif comprenant une cellule d'écoulement électrochimique comprenant :
• un corps ayant un trajet d'écoulement, le trajet d'écoulement ayant une entrée et une sortie ;
• une électrode de travail en communication fluidique avec le trajet d'écoulement ; et
• une électrode auxiliaire ;
dans lequel l'électrode de travail comprend un composant ou une couche qui contient au moins 20 % en poids de titane sous la forme de l'élément titane, de substances contenant du titane et/ou d'alliages contenant du titane ; et
dans lequel la sortie de la cellule est reliée à un dispositif de détection électrochimique (ECD), un spectromètre RMN ou un spectromètre de masse (SM).

10. Dispositif selon la revendication 9, dans lequel la cellule électrochimique est une cellule d'écoulement électrochimique en couche mince avec un volume de travail de 0,1 µl à 1 ml et une distance entre l'électrode de travail et l'électrode auxiliaire de 10 à 150 µm.

11. Dispositif selon la revendication 9 ou 10, dans lequel l'électrode auxiliaire comprend un matériau d'électrode choisi parmi le titane, le graphite, le platine et des combinaisons de ceux-ci.

12. Dispositif selon l'une quelconque des revendications 9 à 11, dans lequel l'électrode de travail comprend l'élément titane.
